# EUROPEAN PATENT APPLICATION

(11) **EP 1 219 320 A2**
(43) Date of publication of application: **03.07.2002**
(21) Application number: 01130393.0
(22) Date of filing: 20.12.2001
(51) Int. Cl.: A61N 1/32, A61N 2/00

(54) **Apparatus for electrotherapy and/or magnetotherapy**

(30) Priority: 22.12.2000 IT UD000226
(71) Applicant: Elettra 80 S.r.l., 31020 S. Maria Del Piave, (TV) (IT)
(72) Inventor: Borin, Giovanni, 31020 S. Maria del Piave (TV) (IT); Zotta, Paolo, 36060 Romano d'Ezzelino (VI) (IT); Giacomazzi, Daniele, 31020 S. Maria del Piave (TV) (IT)
(74) Representative: Petraz, Gilberto Luigi

(57) **Abstract**

Apparatus for electrotherapy and/or magnetotherapy (10) comprising a plurality of electrodes and electronic control means (21), able to generate and send to the electrodes electric signals having a variable period (p) and intensity (V), wherein the electronic control means (21) are able to sub-divide the period (p) into a plurality of unitary temporal segments, and moreover data introduction means (16) are connected to the electronic control means (21) to allow the user to choose and to set for each of the segments an intensity (V) of the corresponding electric signal comprised between a maximum value (VMAX) and a minimum value (VMIN), so that the wave shape of the electric signal applied to each electrode can be selected and programmed by the user.

## Description

### FIELD OF THE INVENTION

The invention concerns an apparatus for electrotherapy and/or magnetotherapy, provided with a plurality of electrodes able to be applied to particular parts of a human body, or another living being, depending on the therapy which the patient has to do, in order to transmit electric and/or electromagnetic impulses, according to a sequence and intensity which can be programmed and set directly by the patient by means of a console and with the help of a screen displaying information on the chosen therapy and on the settings made.

### BACKGROUND OF THE INVENTION

The state of the art includes apparatuses, also portable apparatuses, which allow to make applications either of electrotherapy alone or of magnetotherapy alone.

Such conventional apparatuses are normally provided with a plurality of electrodes to be applied on particular parts of the human body, to transmit electric or corresponding electromagnetic impulses, the intensity and duration of which can be regulated by means of knobs which command an electric potentiometer and a timer.

Conventional apparatuses, however, are set in a rigid manner and, apart from the intensity of the current reaching the electrodes, and the period of each impulse, which as we have seen can be regulated, always generate a rectangular shaped wave of fixed amplitude. The close succession of several waves creates an electro-medical application program. All the programs inserted, however, cannot be modified in any way.

This is a limit and disadvantage, especially if we think that the therapies to be applied are different according to the pathologies to be treated; moreover, they are almost always different from one patient to the other and, even for the same patient, they can be different from each other according to the phase of application of the same therapy.

The present Applicant has devised and embodied the apparatus for electrotherapy and/or magnetotherapy according to the invention to overcome the above-mentioned shortcomings and to obtain further advantages.

### SUMMARY OF THE INVENTION

The apparatus for electrotherapy and/or magnetotherapy according to the invention is set forth and characterized in the main claim, while the dependent claims describe other innovative features of the invention.

One purpose of the invention is to achieve an apparatus which will allow to perform both electrotherapy and magnetotherapy treatments on any living being and wherein the intensity and the wave shape of the electric signal applied to each electrode can be modulated and programmed by the user himself, in a personalized fashion according to the specific pathology to be treated. By user we mean the person who uses and commands the functioning of the apparatus, irrespective of the living being on which the electrodes are applied, while they can always be applied on the user himself.

In accordance with this purpose, the apparatus according to the invention comprises electronic control means, associated with data introduction means and display means, by means of which the user can set and visually control the parameters which determine the wave shape and the amplitude of the electric signal to be associated with each electrode; electronic memory means are provided to memorize the above-mentioned data set by the user to reproduce, automatically and as chosen by the user himself, the sequence of therapeutic program selected.

The apparatus according to the invention comprises a structure which contains an electronic control circuit and on which a console is mounted provided with function keys and a display.

The display, according to the therapy chosen, also shows the points at which the electrodes are to be arranged in order to have a more efficacious result.

The apparatus is advantageously inserted into a portable case and functions by means of an incorporated battery.

### BRIEF DESCRIPTION OF THE DRAWINGS

These and other characteristics of the invention will be clear from' the following description of some preferential forms of embodiment, given as a non-restrictive example, with reference to the attached drawings wherein:
- Fig. 1: is a three-dimensional view of an apparatus for electrotherapy and/or magnetotherapy according to the invention;
- Fig. 2: is a plane view of a console of the apparatus in Fig. 1;
- Fig. 3: is a block diagram of the electric circuit of the apparatus in Fig. 1;
- Fig. 4: is a flow chart showing some working steps of the apparatus in Fig. 1, representing some information, in graphic and alphanumeric form, which can appear on the screen of the console in Fig. 2;
- Fig. 5: is a first enlarged detail of Fig. 4;
- Fig. 6: is a second enlarged detail of Fig. 4.

### DETAILED DESCRIPTION OF A PREFERRED EMBODIMENT

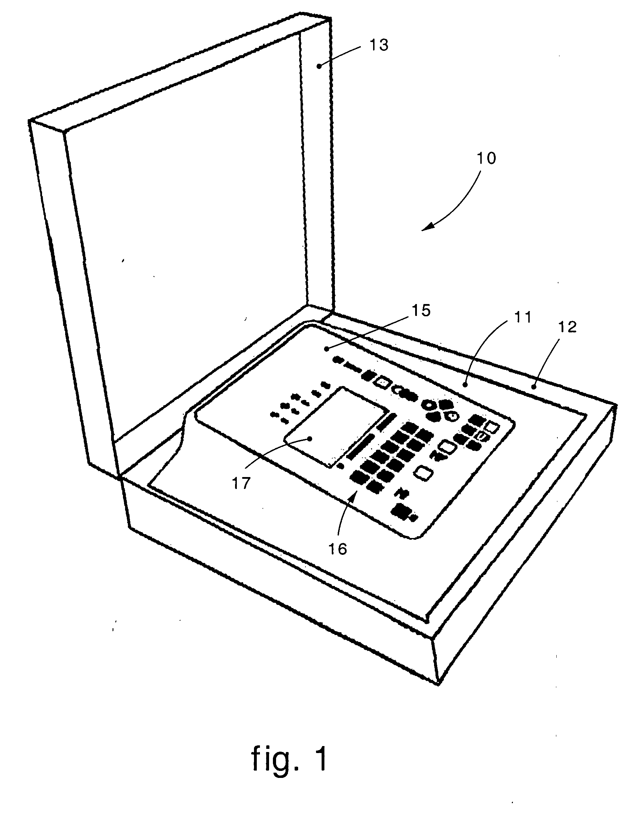

With reference to Fig. 1, an apparatus 10 for electrotherapy and/or magnetotherapy according to the invention comprises a structure 11 made of plastic material, shaped so as to remain housed inside a portable case 12 provided with a lid 13.

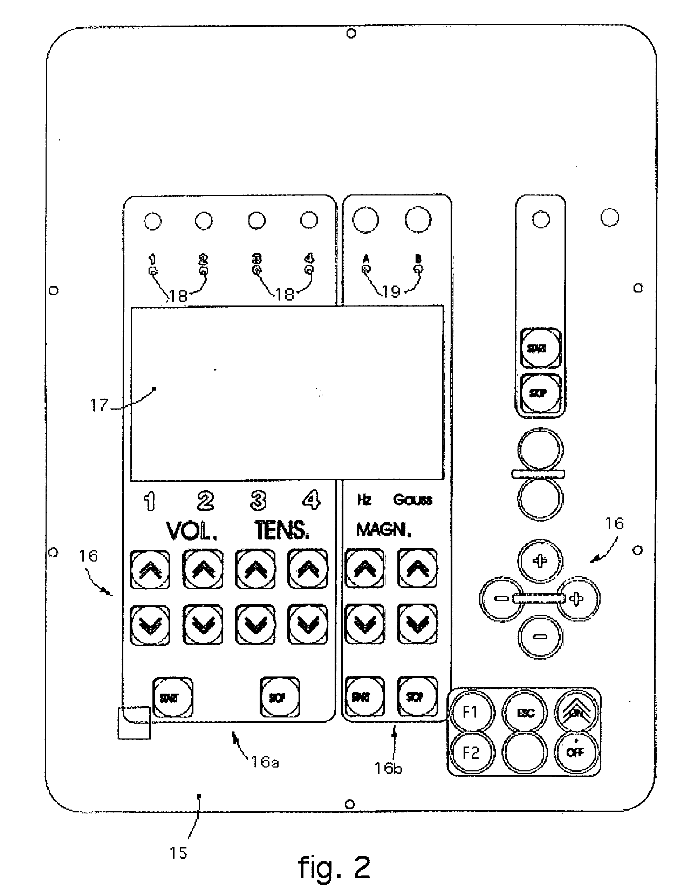

The structure 11 comprises a substantially rectangular console 15 (Figs. 1 and 2), with a plurality of keys 16, a screen or display 17, for example a liquid crystal display, four outlets 18 with a female connector, associated with the numbers 1, 2, 3 and 4, to which four electrodes, of a conventional type and not shown in the drawings, are connected for the electrotherapy applications, and two outlets 19 with a female connector, associated with the letters A and B, to which two electrodes, also of a conventional type and not shown in the drawings, are connected for the magnetotherapy applications.

To be more exact the console 15 comprises keys 16a dedicated to electrotherapy, and keys 16b dedicated to magnetotherapy.

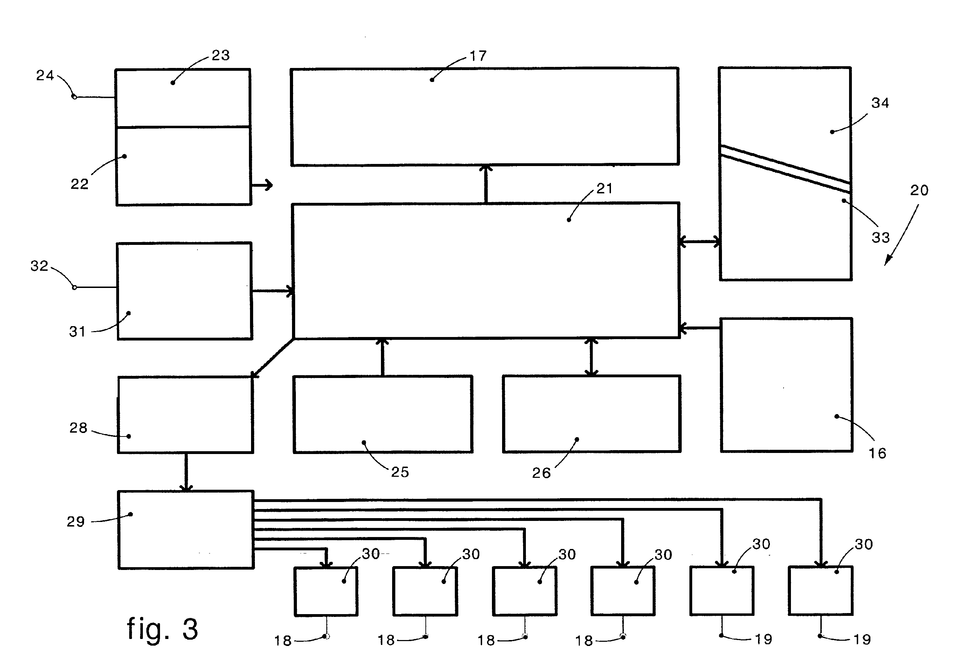

Inside the structure 11 there is an electronic circuit 20 (Fig. 3) comprising a microprocessor or microcontroller 21 connected to the keys 16 of the console 15 and to the display 17. The circuit 20 also comprises a power supply 22, consisting of a battery and able to generate a low-voltage continuous current of about 12V, to which is associated a battery-charging apparatus 23 able to be connected to an external AC electric source 24 by means of an insulating transformer.

A Read Only Memory (ROM) 25, in which the management programs of the entire apparatus 10 are able to be memorized, and a non-volatile memory (EEPROM) 26 in which to save the data, are also connected to the microcontroller 21.

Six circuits 30 to command the modulated tension between about +5V to -5V are also connected, by means of a 12 bit analog digital converter 28 and a multiplexer 29, to the microcontroller 21. Each of the circuits 30 is connected to a connector 18 or 19.

The circuit 20 also comprises a test device 31, which can be connected to an external search probe 32 of a conventional type, to generate a corresponding electric signal for the microcontroller 21.

A serial port 33 for the input/output of data, opto-insulated, is connected to the microcontroller 21 and can be selectively connected to a standard serial port 34 for personal computers, for example of the RS232 type.

The circuit 20 is able to generate, for each outlet 18 or 19, an electric signal having a set period (p) and a wave shape which can be programmed by the user of the apparatus 10, that is, by the doctor or the patient himself on which the electrotherapy or magnetotherapy is to be applied.

To be more exact, each elementary signal output from the connectors 18 or 19 is formed by a plurality of segments, for example 28, each of which has a pre-set or unitary duration, determined by the timer inside the microcontroller 21, for example 285 µs.

The amplitude of each segment can be set between a maximum value VMAX, for example +5V, and a minimum value VMIN, for example -5V, by means of the keys 16 of the console 15, so that the current applied to each electrode connected to the connectors 18 and 19 will have any wave shape whatsoever, chosen and programmed, in personalized manner, by each user.

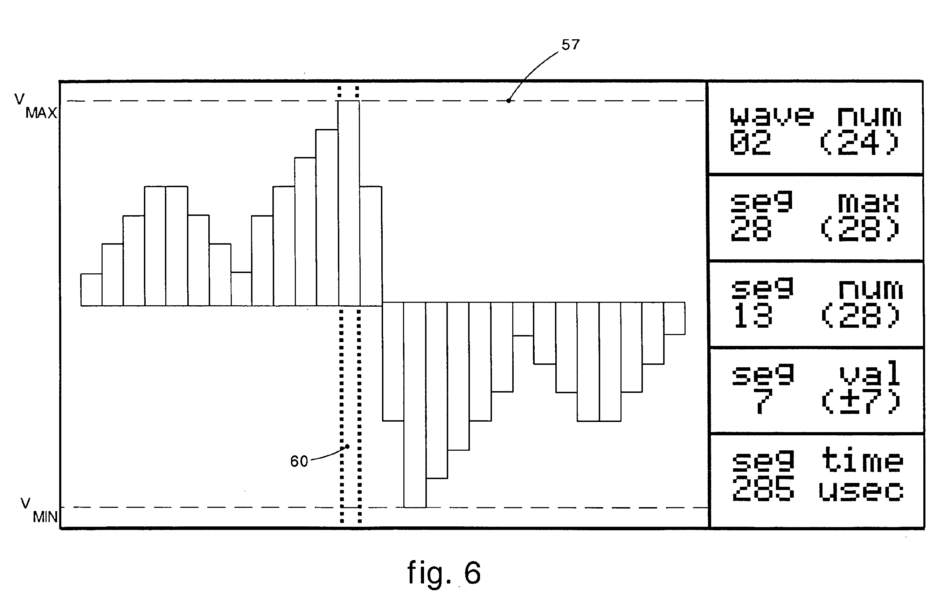

Fig. 6 shows an example of a wave shape of a signal output from a connector 18, as shown on the display 17.

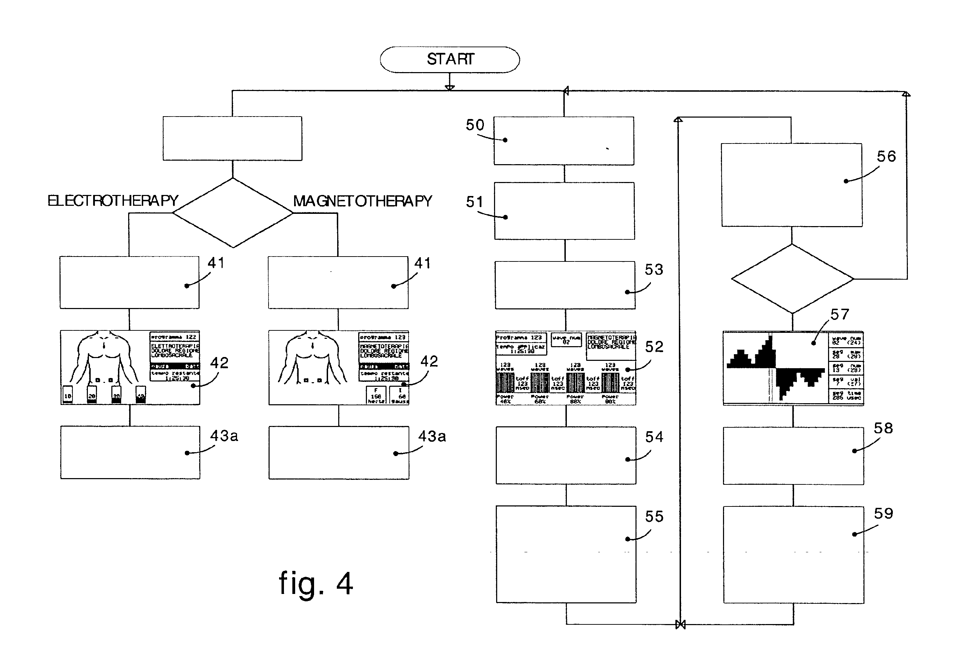

With reference to Fig. 4, the apparatus 10 as described heretofore functions as follows.

When the apparatus 10 is switched on, a message 40 appears on the display 17 indicating the state of the apparatus 10 and the user is invited to select the program and therapy he wants, either electrotherapy or magnetotherapy, in the case of a program already set and memorized in the memory 27. The setting and memorization of a new therapy program will be explained later.

The choice is made by actuating the keys 16a or 16b and possibly by recalling a previously set program to which an alphabetic, numeric or alphanumeric identification code 41 has been assigned. Once the therapy program has been identified, a figure 42 appears on the display 17 which invites the user to position the electrodes connected to the outlets 18 or 19 in a particular manner, as indicated in the image 42.

The images 42 shown in Fig. 4 refer, as an a example, to therapies concerning a pain localized in the lumbo-sacral region.

In the case of magnetotherapy, the user can further regulate the intensity of the signal applied (gauss) and the wave frequency (Hz) used.

The therapy (43a, 43b) is started by pressing the corresponding key START of the section 16a or 16b.

If the therapy is to be programmed personally, the following steps are performed.

In the first step 50 the programming enablement data is set; then the key ESC is pressed for several seconds until an acoustic signal, associated with the microcontroller 21 and not shown in the drawings, indicates that enablement has taken place (step 51).

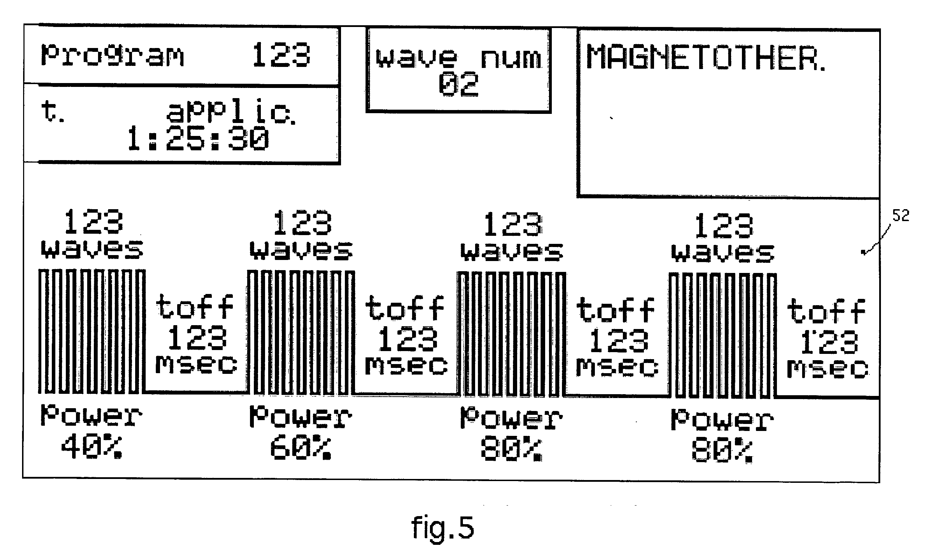

Then, the key F2 (down) is pressed to enable the "program" page, which makes an image 52 (Figs. 4 and 5) appear on the display 17; the image 52 shows the values of number, time and intensity associated with the wave shape of the signal which will then be applied to the corresponding electrode.

With the cursor keys positioned in the desired field 16a or 16b, it is possible to modify the relative values (step 54). The data is subsequently saved or remodified (step 55), before setting a new program.

With a following step 56, pressing a key F1 (up), it is possible to close the programming cycle and return to the first page, or, by pressing a key F2 (down), it is possible to enable the display of the image 57 (Figs. 4 and 6).

Like the steps 54 and 55, with the cursor keys positioned in the corresponding section 16a or 16b, it is possible to modify the relative values (step 58), segment by segment, and the data is saved or remodified (step 59). The segment 60 being operated on is displayed on the display 17 (Fig. 6).

It is clear however that modifications and/or additions of parts can be made to the apparatus for electrotherapy and/or magnetotherapy 10 as described heretofore without departing from the spirit and scope of the invention.

It is also clear that, although the invention has been described with reference to a specific example, a person of skill in the art shall certainly be able to achieve many other equivalent forms of the apparatus, all of which shall come within the field and scope of this invention.

## Claims

1. Apparatus for electrotherapy and/or magnetotherapy comprising a plurality of electrodes and electronic control means (21) able to generate and send to said electrodes electric signals having a variable period (p) and intensity (V), **characterized in that** said electronic control means (21) are able to sub-divide said period (p) into a plurality of unitary temporal segments and that data introduction means (16) are connected to said electronic control means (21) to allow the user to choose and to set for each of said segments an intensity (V) of the corresponding electric signal between a maximum value (VMAX) and a minimum value (VMIN), so that the wave shape of the electric signal applied to each electrode can be selected and programmed by the user.

2. Apparatus as in claim 1, **characterized in that** display means (17) are provided to display and allow the user to control the settings of said data and of the associated wave shape resulting.

3. Apparatus as in claim 2, **characterized in that** electronic memory means (26) are provided to memorize said data set to reproduce automatically, and according to the choice of the user himself, the sequence of therapeutic program selected.

4. Apparatus as in claim 1, **characterized in that** said display means (17) are also able to display the points at which said electrodes must be arranged for a more efficacious result of the chosen therapy.

5. Apparatus as in claim 2, **characterized in that** said display means (17) and said data introduction means (16) are mounted on a structure (11) inside which said electronic control means (21) are arranged, said structure (11) being inserted in a portable case (12).

6. Apparatus as in claim 1, **characterized in that** said electronic control means comprise a microcontroller (21) connected to a power supply (22), consisting of at least a battery able to supply a continuous current with a tension of about 12V.

7. Apparatus as in claim 6, **characterized in that** a Read Only Memory (25), in which the management programs of the entire apparatus are able to be memorized, and a data memory (26) are also connected to said microcontroller (21).

8. Apparatus as in claim 6, **characterized in that** a plurality of circuits (30) is also connected to said microcontroller (21) by means of a digital converter (28) and a multiplexer (29) to modulate said tension, each of said circuits (30) being connected to a connector (18, 19) on which one of said electrodes is able to be connected.

9. Apparatus as in claim 6, **characterized in that** a test device (31) is able to be connected to an external search probe (32) to generate a corresponding electric signal for said microcontroller (21).

10. Apparatus as in claim 6, **characterized in that** a serial port (33) for the input/output of data is connected to said microcontroller (21) and can be selectively connected to a standard serial port (34) for personal computers.
